# FASCICULE DE BREVET EUROPEEN

(11) **EP 2 225 241 B1**
(45) Date de publication et mention de la délivrance du brevet: **14.09.2011**
(21) Numéro de dépôt: 08872826.6
(22) Date de dépôt: 31.12.2008
(51) Int. Cl.: C07D 471/04, A61K 31/437, A61P 25/00, A61P 35/00, A61P 19/00, A61P 29/00, C07D 233/70, C07D 213/74, C07D 213/30, C07D 233/74, A61P 19/10

(54) **Dérivés de N-phényl-imidazo-(1,2-A)-pyridine2-carboxamides, leur préparation et leur application en thérapeutique**
Derivate aus N-phenyl-imidazo-(1,2-A)-pyridin-2-carboxamiden, ihre Herstellung sowie ihre therapeutische Anwendung
Derivatives of N-phenyl-imidazo-(1,2-A)-pyridine-2-carboxamides, preparation thereof and therapeutic application thereof

(30) Priorité: 02.01.2008 FR 0800002
(43) Date de publication de la demande: 08.09.2010
(73) Titulaire: SANOFI, 75013 Paris (FR)
(72) Inventeur: PEYRONEL, Jean-François, F-75013 Paris (FR)
(74) Mandataire: Le Coupanec, Pascale A.M.P.
(86) Numéro de dépôt international: PCT/FR2008/001833
(87) Numéro de publication internationale: WO 2009/106748

(56) Documents cités:
- WO-A-2006/067446
- WO-A-2008/003856
- FR-A- 2 638 161
- US-A1- 2005 261 315
- DATABASE REGISTRY [Online] CAS; 1 mars 2007 (2007-03-01), XP002493975 extrait de STN Database accession no. 924038-88-0 (RN)
- DATABASE REGISTRY [Online] CAS; 1 mars 2007 (2007-03-01), XP002493976 extrait de STN Database accession no. 924031-00-5 (RN)
- DATABASE REGISTRY [Online] CAS; 1 mars 2007 (2007-03-01), XP002493977 extrait de STN Database accession no. 924102-15-8 (RN)
- DATABASE REGISTRY [Online] CAS; 1 mars 2007 (2007-03-01), XP002493978 extrait de STN Database accession no. 924040-86-8 (RN)
- DATABASE REGISTRY [Online] CAS; 28 août 2001 (2001-08-28), XP002493979 extrait de STN Database accession no. 353258-70-5 (RN)
- DATABASE REGISTRY [Online] CAS; 28 août 2001 (2001-08-28), XP002493980 extrait de STN Database accession no. 353258-68-1 (RN)

## Description

La présente invention se rapporte à des dérivés d'imidazo[1,2-*a*]pyridine-2-carboxamides, à leur préparation et à leur application en thérapeutique dans le traitement ou la prévention de maladies impliquant les récepteurs nucléaires Nurr-1, aussi appelés NR4A2, NOT, TINUR, RNR-1, et HZF3.

La présente invention a pour objet les composés de formule (I) : 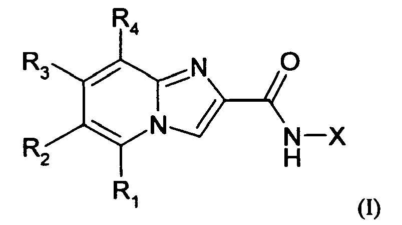 dans laquelle :
X représente un groupe phényle substitué par un cyano, un (C₁-C₆)alcoxycarbonyle, un (C₁-C₆)alcoxy substitué par un ou plusieurs halogènes ou (C₁-C₆)alkyle substitué par un ou plusieurs halogènes, le groupe phényle étant éventuellement substitué une seconde fois par un halogène;
R₁ représente un atome d'hydrogène, un halogène, un groupe (C₁-C₆)alcoxy, un groupe (C₁-C₆)alkyle, un groupe NRaRb; les groupes alkyles et alcoxy pouvant éventuellement être substitués par un ou plusieurs halogène, hydroxy, amino, ou groupe (C₁-C₆)alcoxy ;
R₂ représente l'un des groupes suivants :
   - un atome d'hydrogène,
   - un groupe (C₁-C₆)alkyle éventuellement substitué par un ou plusieurs groupes choisis indépendamment les uns des autres parmi un hydroxy, un halogène, un amino, un groupe NRaRb, un groupe (C₁-C₆)alcoxy, un groupe phényle,
   - un groupe (C₁-C₆)alcoxy éventuellement substitué par un ou plusieurs groupes choisis indépendamment les uns des autres parmi hydroxy, halogène, amino, groupe NRaRb,
   - un groupe (C₂-C₆)alcényte,
   - un groupe (C₁-C₆)alcynyle,
   - un groupe -CO-R₅,
   - un groupe -CO-NR₆R₇,
   - un groupe -CO-O-R₈,
   - un groupe -NR₉-CO-R₁₀,
   - un groupe -NR₁₁R₁₂,
   - un groupe -N=CH-NRaRb,
   - un atome d'halogène,
   - un groupe cyano, nitro, hydroxyiminoalkyle, alcoxyiminoalkyle,
   - un groupe (C₁-C₆)alkylthio,
   - un groupe (C₁-C₆)alkylsulfinyle,
   - un groupe (C₁-C₆)alkylsulfonyle,
   - un groupe (((C₁-C₁)alkyl)₃)silyléthynyle,
   - un groupe -SO₂-NR₉R₁₀,
   - un groupe phényle éventuellement substitué par un ou plusieurs groupes choisis indépendamment les uns des autres parmi les atomes ou groupes suivants : halogène, (C₁-C₆)alcoxy, cyano, NRaRb, -CO-R₅, -CO-NR₆R₇, -CO-O-R₈, un groupe (C₁-C₆)alkyle éventuellement substitué par un ou plusieurs hydroxy ou NRaRb ;
R₃ représente un atome d'hydrogène, un groupe (C₁-C₆)alkyle, un groupe (C₁-C₆)alcoxy ou un atome d'halogène ;
R₄ représente un atome d'hydrogène, un groupe (C₁-C₄)alkyle, un groupe (C₁-C₄)alcoxy ou un atome de fluor ;
R₅ représente un atome d'hydrogène, un groupe phényle ou un groupe (C₁-C₆)alkyle ;
R₆ et R₇, identiques ou différents, représentent un atome d'hydrogène ou un groupe (C₁-C₆)alkyle ou forment avec l'atome d'azote un cycle de 4 à 7 chaînons incluant éventuellement un autre hétéroatome choisi parmi N, O ou S ;
R₈ représente un groupe (C₁-C₆)alkyle ;
R₉ et R₁₀, identiques ou différents, représentent un atome d'hydrogène ou un groupe (C₁-C₆)alkyle ;
R₁₁ et R₁₂ identiques ou différents, représentent un atome d'hydrogène ou un groupe (C₁-C₆)alkyle ou forment avec l'atome d'azote un cycle de 4 à 7 chaînons incluant éventuellement un autre hétéroatome choisi parmi N, O ou S ;
Ra et Rb représentent, indépendamment l'un de l'autre, un atome d'hydrogène, un groupe (C₁-C₆)alkyle ou forment avec l'atome d'azote un cycle de 4 à 7 chaînons ;
à l'exception des composés où R₁ représente un groupe méthyle ou R₂ représente un atome de chlore ou R₃ représente un groupe méthyle,
à l'état de base ou de sel d'addition à un acide,
à l'exception des composés :
   N-(3-chloro-4-cyanophényl)-imidazo[1,2-a]pyridine-2-carboxamide,
   3-({[imidazo[1,2-a]pyridine-2-yl]carbonyl}amino)benzoate de méthyle,
   2-({[imidazo[1,2-a]pyridine-2-yl]carbonyl}amino)benzoate de méthyle,
   N-[2-(difluorométhoxy)phényl]-imidazo[1,2-a]pyridine-2-carboxamide,
   N-[3-(trifluorométhoxy)phényl]-6-(diméthylamino)imidazo[1,2-a]pyridine-2-carboxamide,
   N-[3-(difluorométhoxy)phényl]-6-(diméthylamino)imidazo[1,2-a]pyridine-2-carboxamide, et
   N-[3-(trifluorométhyl)phényl]-6-(diméthylamino)imidazo[1,2-a]pyridine-2-carboxamide.

On connaît de WO 2008/003856 des composés ayant une activité sur les récepteurs précités. Les composés N-[3-trifluorométhoxy)phényl]-6-(diméthylamino)imidazo[1,2-a]pyridine-2-carboxamide (exemple 76), N-[3-(difluorométhoxy)phényl]-6-(diméthylamino)imidazo[1,2-a]pyridine-2-carboxamide (exemple 82), et N-[3-(trifluorométhyl)phényl]-6-(diméthylamino)imidazo[1,2-a]pyridine-2-carboxamide (exemple 83), sont spécifiquement exclus de la formule générale (1) selon l'invention.

On connaît également du document US 2005/261315, des dérivés de composés amides qui modulent l'activité des kinases.

La demande WO 2006/067446 décrit des dérivés de composés pyridine carboxamide, inhibiteurs de protéines kinases.

En outre, les composés (3-chloro-4-cyanophényl)-imidazo[1,2-a]pyridine-2-carboxamide (Database accession No. 924038-88-0), 3-({[imidazo[1,2-a]pyridine-2-yl]carbonyl}amino)benzoate de méthyle (No. 924031-00-5), 2-({[imidazo[1,2-a]pyridine-2-yl]carbonyl}amino)benzoate de méthyle (No. 924102-15-8) et N-[2-(difluorométhoxy)phényl]-imidazo[1,2-a]pyridine-2-carboxamide (No. 924040-86-8), pour lesquels aucune activité pharmacologique ou thérapeutique n'a été démontrée, sont également spécifiquement exclus de ladite formule (I) selon l'invention.

Le composés N-(3-cyanophényl)-{6-trifluorométhyl-8-chloro-imidazo[1,2-a]pyridine}-2-carboxamide (Database accession No. 353258-70-5) et 4-({6-trifluorométhyl-8-chloro-[imidazo[1,2-alpyridine-2-yl]carbonyl}amino)benzoate de méthyle (Database accession No. 353258-68-1), pour lesquels aucune activité pharmacologique ou thérapeutique n'a été démontrée, sont également connus. Cependant, les composés de la présente invention se distinguent de ces composés par l'impossibilité d'avoir un substituant chlore en position 8 de l'hétérocycle.

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.

Les composés de formule (I) peuvent exister à l'état de bases ou de sels d'addition à des acides. De tels sels d'addition font partie de l'invention.

Ces sels peuvent être préparés avec des acides pharmaceutiquement acceptables, mais les sels d'autres acides utiles, par exemple, pour la purification ou l'isolement des composés de formule (I) font également partie de l'invention.

Les composés de formule (I) peuvent également exister sous forme d'hydrates ou de solvats, à savoir sous forme d'associations ou de combinaisons avec une ou plusieurs molécules d'eau ou avec un solvant. De tels hydrates et solvats font également partie de l'invention.

Dans le cadre de la présente invention, on entend par :
- un atome d'halogène : un fluor, un chlore, un brome ou un iode ;
- un groupe alkyle : un groupe aliphatique saturé linéaire, ramifié ou cyclique, éventuellement substitué par un groupe alkyle saturé linéaire, ramifié ou cyclique. A titre d'exemples, on peut citer les groupes méthyle, éthyle, propyle, isopropyle, butyle, isobutyle, tertbutyle, cyclopropyle, cyclobutyl, cyclopentyle, cyclohexyle, méthylcyclopropyl etc ;
- un groupe alcényle : un groupe aliphatique mono- ou poly-insaturé, linéaire ou ramifié, comprenant par exemple une ou deux insaturations éthyléniques ;
- un groupe alcoxy : un radical -O-alkyle où le groupe alkyle est tel que précédemment défini ;
- un groupe alcynyle : un groupe aliphatique mono- ou poly-insaturé, linéaire ou ramifié, comprenant par exemple une ou deux insaturations éthylyniques.

Selon un autre de ses aspects, la présente invention a pour objet les composés de formule (I) dans laquelle X, R₁ à R₄ sont tels que définis précédemment, étant entendu qu'au moins l'un des R₁, R₂, R₃ et R₄ est différent d'un atome d'hydrogène, à l'état de base ou de sel d'addition à un acide,
à l'exception des composés où R₁ représente un groupe méthyle ou R₂ représente un atome de chlore ou R₃ représente un groupe méthyle,
à l'exception des composés :
N-[3-(trifluorométhoxy)phényl]-6-(diméthylamino)imidazo[1,2-a]pyridine-2-carboxamide,
N-[3-(difluorométhoxy)phényl]-6-(diméthylamino)imidazo[1,2-a]pyridine-2-carboxamide, et
N-[3-(trifluorométhyl)phényl]-6-(diméthylamino)imidazo [1,2-a]pyridine-2-carboxamide.

Selon un autre de ses aspects, la présente invention a pour objet les composés de formule (I) dans laquelle X, R₁ à R₄ sont tels que définis précédemment, étant entendu qu'au moins l'un des R₁, R₂, R₃ et R₄ est différent d'un atome d'hydrogène, à l'état de base ou de sel d'addition à un acide,
à l'exception des composés où R₁ représente un groupe méthyle ou R₂ représente un atome de chlore ou R₃ représente un groupe méthyle,
à l'exception des composés pour lesquels R₂ représente un groupe N-diméthyle et X représente :
- un groupe phényle substitué par un groupe méthoxy lui-même substitué par deux ou trois atomes de fluor, ou
- un groupe phényle substitué par un groupe méthyle lui-même substitué par trois atomes de fluor.

Selon un autre de ses aspects, la présente invention a également pour objet un premier groupe de composés de formule (I) dans laquelle :
X représente un groupe phényle substitué par un cyano, un (C₁-C₆)alcoxycarbonyl, un (C₁ C₆)alcoxy substitué par plusieurs halogènes, ledit groupe phényle étant éventuellement substitué une seconde fois par un halogène ;
R₂ représente un groupe -NR₁₁R₁₂ ou un groupe phényle substitué par un groupe (C₁-C₆)alkyle lui-même substitué par un groupe hydroxy ;
R₁, R₃ et R₄ représentent un atome d'hydrogène ;
R₁₁, et R₁₂ identiques ou différents, représentent un groupe (C₁-C₆)alkyle ;
à l'état de base ou de sel d'addition à un acide,
à l'exception des composés où R₁ représente un groupe méthyle ou R₂ représente un atome de chlore ou R₃ représente un groupe méthyle,
à l'exception des composés :
   *N*-[3-(trifluorométhoxy)phényl)-6-(diméthylamino)imidazo[1,2-*a*]pyridine-2-carboxamide et
   *N*-[3-(difluorométhoxy)phényl)-6-(diméthylamino)imidazo[1,2-*a*]pyridine-2-carboxamide.

Selon encore un autre de ses aspects, la présente invention a pour objet un second groupe de composés de formule (I) dans laquelle :
X représente un groupe phényle substitué par un cyano, CO₂Me, OCHF₂, éventuellement substitué une seconde fois par un atome de fluor ;
R₁, R₃ et R₄ représentent un atome d'hydrogène ;
R₂ représente un groupe N-diméthyle, ou un groupe phényle substitué par un groupe hydroxyméthyle ;
à l'état de base ou de sel d'addition à un acide,
à l'exception du N-[3-(difluorométhoxy)phényl]-6-(diméthylamino)imidazo[1,2,a]pyridine-2-carboxamide.

Parmi les composés de formule (I) objets de l'invention, on peut notamment citer les composés suivants :
- *N*(3-cyano-5-fluorophényl)-6-[3-(hydroxyméthyl)phényl]imidazo[1,2-*a*]pyridine-2-carboxamide
- *N*-(3-cyanophényl)-6-(diméthylamino)imidazo[1,2-*a*]pyridine-2-carboxamide
- 3-({[6-(Diméthylamino)imidazc[1,2-*a*]pyridin-2-yl]carbonyl}amino)benzoate de méthyle
- *N*-[3-(difluorométhoxy)phényl]-6-[3-(hydroxyméthyl)phényl]imidazo[1,2-*a*]pyridine-2-carboxamide
- *N*-(3-cyanophényl)-6-[3-(hydroxyméthyl)phényl]imidazo[1,2-*a*]pyridine-2-carboxamide
- *N*-(4-cyano-2-fluorophényl)-6-(diméthylamino)imidazo[1,2-*a*]pyridine-2-carboxamide
- *N*-(2-cyano-3-fluorophényl)-6-(diméthylamino)imidazo[1,2-*a*]pyridine-2-carboxamide
- *N*-(2-cyano-3-fluorophényl)-6-[3-(hydroxyméthyl)phényl]imidazo[1,2-*a*]pyridine-2-carboxamide
- *N*-(3-cyano-5-fluorophényl)-6-(diméthylamino)imidazo[1,2-*a*]pyridine-2-carboxamide, et leurs sels d'addition à un acide.

Conformément à l'invention, on peut préparer les composés de formule générale (I) selon le procédé décrit dans le schéma 1.

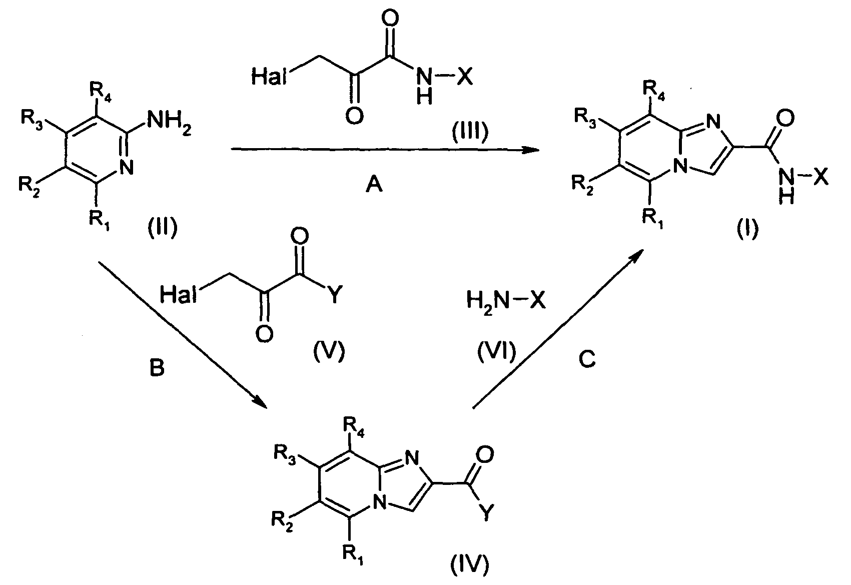

La voie A consiste à préparer les 2-amino-pyridines de formule (II) selon les méthodes connues de l'homme du métier et à former le cycle imidazo [1,2-a]pyridine par condensation sur un dérivé de 2-oxo-*N-*aryl-propionamide (III) dans lequel Hal représente un atome de chlore, de brome ou d'iode et X est défini comme précédemment par analogie avec les méthodes décrites par J-J. Bourguignon et coll. dans Aust. J. Chem., 50, 719 (1997) et par J.G. Lombardino dans J. Org. Chem., 30, 2403 (1965) par exemple. Les dérivés halogènés de 2-oxo-*N*-aryl-propionamide (III) peuvent être obtenus selon la méthode décrite par R. Kluger et coll. dans J. Am. Chem. Soc., 106, 4017 (1984).

La seconde voie de synthèse B, C consiste à coupler un acide imidazopyridine-2-carboxylique ou l'un de ses dérivés, de formule (IV) dans laquelle Y représente un groupe hydroxy, un atome d'halogène ou un groupe (C₁-C₆)alcoxy, avec une arylamine X-NH₂ (VI) dans laquelle X est défini comme précédemment selon des méthodes connues de l'homme du métier. Ainsi l'acide peut être préalablement converti en l'un de ses dérivés réactifs tel que halogénure d'acide, anhydride, anhydride mixte ou ester activé puis mis en réaction avec l'amine (VI) en présence d'une base telle que la diisopropyléthylamine, la triéthylamine ou la pyridine, dans un solvant inerte tel que le THF, le DMF ou le dichlorométhane. Le couplage peut également être réalisé en présence d'un agent de couplage tel que CDI, EDCI, HATU ou HBTU dans les mêmes conditions sans isoler d'intermédiaire réactif. Alternativement on peut faire réagir l'amine (VI) avec un ester de l'acide de formule (IV) en présence d'un catalyseur tel que le triméthylaluminium selon la méthode de Weinreb, S. et coll (Tet. Lett., 18, 4171 (1977)) ou le terbutylate de zirconium. Les acides imidazopyridine-2-carboxyliques et leurs dérivés de formule (IV) peuvent être obtenus en condensant les 2-aminopyridines appropriées sur un ester de l'acide 3-halogèno-2-oxo-propionique selon la méthode décrite par J.G. Lombardino dans J. Org. Chem., 30(7), 2403 (1965), puis en déprotégeant l'ester en acide et convertissant le cas échéant l'acide en l'un de ses dérivés.

Les produits de formule (I) et leurs précurseurs de formule (II) ou (IV), peuvent être soumis, si désiré et si nécessaire, pour obtenir des produits de formule (I) ou être transformés en d'autres produits de formule (I) à l'une ou plusieurs des réactions de transformations suivantes, dans un ordre quelconque :
a) une réaction d'estérification ou d'amidification de fonction acide,
b) une réaction d'amidification de fonction amine,
c) une réaction d'hydrolyse de fonction ester en fonction acide,
d) une réaction de transformation de fonction hydroxyle en fonction alcoxy,
e) une réaction d'oxydation de fonction alcool en fonction aldéhyde ou cétone,
f) une réaction de transformation des fonctions aldéhyde ou cétone en fonction alcool par réduction ou action d'un organométallique tel qu'un organomagnésien,
g) une réaction de conversion des fonctions aldéhyde ou cétone en dérivé oxime,
h) une réaction de transformation de radical nitrile en fonction aldéhyde,
i) une réaction de transformation de radical nitrile en fonction cétone,
j) une réaction d'oxydation de groupe alcènyle en fonction aldéhyde ou cétone,
k) une réaction d'oléfination de fonction aldéhyde ou cétone en groupe alcènyle,
l) une réaction de deshydratation de groupe hydroxyalkyle en groupe alcényle,
m) une réaction d'hydrogénation totale ou partielle de groupe alcényle ou alcynyle en groupe alcényle ou alkyle,
n) une réaction de couplage catalytique d'un dérivé organométallique tel qu'un dérivé du bore, de l'étain ou du silicium avec un dérivé halogèné pour introduire un substituant alkyle, alcènyle, alcynyle ou aryle,
o) une réaction de réduction d'un groupe nitro en groupe amino primaire,
p) une réaction de conversion d'un groupe amino primaire ou secondaire en un groupe amino, secondaire ou tertiaire par amination réductrice ou alkylation,
q) une réaction de conversion d'un groupe amino primaire en un groupe amidine,
r) une réaction d'oxydation de fonction thioéther en fonction sulfoxide ou sulfone,
s) une réaction de protection des fonctions réactives,
t) une réaction d'élimination des groupements protecteurs que peuvent porter les fonctions réactives protégées,
u) une réaction de salification par un acide minéral ou organique ou par une base pour obtenir le sel correspondant,
v) une réaction de dédoublement des formes racémiques en énantiomères,
   lesdits produits de formule (I) ainsi obtenus étant le cas échéant sous toutes les formes isomères possibles racémiques, énantiomères et diastéréoisomères, tautomères.

Dans le schéma 1, les composés de départ et les réactifs, quand leur mode de préparation n'est pas décrit, sont disponibles dans le commerce ou décrits dans la littérature, ou bien peuvent être préparés selon des méthodes qui y sont décrites ou qui sont connues de l'Homme du métier.

Les exemples suivants décrivent la préparation de certains composés conformes à l'invention. Ces exemples ne font qu'illustrer la présente invention. Les numéros des composés exemplifiés renvoient à ceux donnés dans le tableau ci-après, qui illustre les structures chimiques et les propriétés physiques de quelques composés selon l'invention.

### Exemple 1 : N-(3-cyano-5-fluorophényl)-6-[3-(hydroxyméthyl)phényl]imidazo[1,2-a]pyridine-2-carboxamide

A une solution de 55 mg de 3-amino-5-fluorobenzonitrile dans 1 mL de toluène refroidie à 0°C, on ajoute goutte à goutte 253 µL d'une solution 2M de triméthylaluminium dans le toluène puis après retour à 20°C, on ajoute 100 mg de 6-(3-hydroxyméthyl-phényl)-imidazo[1,2-a]pyridine-2-carboxylate d'éthyle. Le mélange réactionnel est chauffé au reflux pendant 30 heures puis refroidi et dilué par 2 mL d'eau, acidifié à pH 3 par de l'acide chlorhydrique N et extrait par 5 mL d'acétate d'éthyle puis 5 mL de dichlorométhane. Les phases organiques réunies sont séchées sur sulfate de sodium, filtrées et concentrées sous pression réduite. Le résidu joint à l'insoluble isolé à l'interface est lavé successivement par de l'éther éthylique, des mélanges éther éthylique-dichlorométhane (1/1) et dichlorométhane-méthanol (99/1) et par du méthanol pour donner 63 mg de *N*-(3-cyano-5-fluorophényl)-6-[3-(hydroxyméthyl)phényl]imidazo[1,2-*a*]pyridine-2-carboxamide sous la forme d'un solide blanc.

### Exemple 2 : N-(3-cyanophényl)-6-(diméthylamino)imidazo[1,2-a]pyridine-2-carboxamide

A une solution de 200 mg d'acide 6-diméthylamino-imidazo[1,2-a]pyridine-2-carboxylique dans 4 mL de *N,N* diméthylformamide on ajoute 147 mg de 3-aminobenzonitrile, 474 mg d'hexafluorophosphate de 1-oxyde de 1-[bis(diméthylamino)méthylène]-1*H*-1,2,3-triazolo[4,5-*b*]pyridinium (HATU), 169 mg de 1-hydroxy-7-azabenzotriazole (HOAt) et 424 µl de diisopropyléthylamine. Le mélange réactionnel est chauffé à 50°C pendant 48 heures, dilué par 3 mL d'eau et 5 mL d'une solution aqueuse saturée d'hydrogénocarbonate de sodium et agité pendant 30 minutes puis extrait par de l'acétate d'éthyle. Les phases organiques réunies sont séchées sur sulfate de sodium, filtrées et concentrées sous pression réduite. Le résidu est purifié par chromatographie sur silice en éluant par un gradient d'hexane, acétate d'éthyle et méthanol (de 60/37/3 à 0/85/15) pour donner 61 mg de *N*-(3-cyanophényl)-6-(diméthylamino)imidazo[1,2-*a*]pyridine-2-carboxamide.

### Exemple 3 : 3-({[6-(Diméthylamino)imidazo[1,2-a]pyridin-2-yl]carbonyl}amino)benzoate de méthyle

A une suspension de 120 mg d'acide 6-diméthylamino-imidazo[1,2-*a*]pyridine-2-carboxylique et 224 mg de chlorhydrate de 1-(3-diméthylaminopropyl)-3-éthylcarbodümide dans 2 mL de pyridine anhydre, placée sous argon, on ajoute 265 mg de 3-aminobenzoate de méthyle. Le mélange réactionnel est agité pendant 48 heures à 50 °C puis concentré à sec sous pression réduite. Le résidu est repris par 5 mL de chloroforme et lavé par 2 mL d'eau. La phase organique est séchée sur sulfate de magnésium et concentrée à sec sous pression réduite. Le résidu est purifié par chromatographie sur silice en éluant par un gradient d'hexane, acétate d'éthyle et méthanol (de 85/15/0 à 0/85/15) pour donner 105 mg de 3-({[6-(diméthylamino)imidazo[1,2-*a*]pyridin-2-yl]carbonyl}amino)benzoate de méthyle.

Les intermédiaires décrits ci-dessous sont utiles à la préparation des composés de la présente invention.

### 6-Diméthylamino-imidazo[1,2-a]pyridine-2-carboxylate d'éthyle

A une solution de 19,05 g de 5-diméthylaminopyridine-2-amine (J. Chem. Soc. Perkin 1, 68 (1973)) dans 380 mL de diméthoxyéthane on ajoute 26,2 mL de bromopyruvate d'éthyle. Le mélange réactionnel est agité à 20°C pendant 6 heures puis après ajout de 380 mL d'éthanol pendant 20 heures au reflux et enfin, après refroidissement, concentré sous pression réduite. Le solide est repris deux fois dans 350 mL d'éther éthylique au reflux et filtré à chaud puis deux fois dans 350 mL d'acétate d'éthyle au reflux et filtré à chaud pour donner 39,66 g de bromhydrate de 6-diméthylamino-imidazo[1,2-*a*]pyridine-2-carboxylate d'éthyle brut. Ce sel est repris dans 800 mL d'eau et traité en agitant vigoureusement par du carbonate de sodium solide jusqu'à atteindre pH 8-9. La phase aqueuse est extraite trois fois par 500 mL de dichlorométhane, les phases organiques rassemblées sont séchées sur sulfate de magnésium, filtrées et concentrées à sec. Le résidu est purifié par chromatographie flash sur une colonne de silice en éluant par des mélanges d'hexane et d'acétate d'éthyle (de 5/1 à 1/1) pour donner 16,7 g de 6-diméthylamino-imidazo[1,2-a]pyridine-2-carboxylate d'éthyle sous la forme d'une huile verte.

Spectre RMN ¹H (DMSO-d6, δ en ppm) : 8,35 (s, 1H), 7,81 (d, J = 2,2, 1H), 7,45 (d, J = 10, 1H), 7,34 (dd, J = 2,4, 10, 1H), 4,27 (q, J = 7,1, 2H), 2,84 (s, 6H), 1,31 (t, J = 7,1, 3H).

### Acide 6-diméthylamino-imidazo[1,2-a]pyridine-2-carboxylique

A une suspension de 16,7 g de 6-diméthylamino-imidazo[1,2-a]pyridine-2-carboxylate d'éthyle dans un mélange de 220 mL de tétrahydrofuranne et 9,5 mL de méthanol, on ajoute à 0°C 107 mL d'une solution aqueuse 2N de lithine. Le mélange réactionnel est ensuite réchauffé à 20°C et agité pendant 4 heures. De l'acide chlorhydrique 2N est ajouté goutte à goutte au mélange réactionnel refroidi à 0°C jusqu'à atteindre un pH de 4-5. Le précipité est filtré et lavé deux fois par 50 mL d'éther éthylique pour donner 14,8 g d'acide 6-diméthylamino-imidazo[1,2-*a*]pyridine-2-carboxylique sous forme d'un solide jaune.

Spectre RMN ¹H (DMSO-d6, δ en ppm) : 8,67 (s, 1H), 8,18 (d, J = 2, 1H), 7,88 (dd, J = 2,4, 10, 1H), 7,75 (d, J = 10, 1H), 2,96 (s, 6H), (1H acide peu visible).

### 6-(3-Hydroxyméthyl-phényl)-imidazo[1,2-a]pyridine-2-carboxylate d'éthyle

A un mélange de 25 g de 6-bromo-imidazo[1,2-*a*]pyridine-2-carboxylate d'éthyle, 13 g d'acide 3-hydroxyméthyl-phénylboronique, 5 g de 2-(dicyclohexylphosphino)biphényle, 1,6 g d'acétate de palladium et 19 g de phosphate de potassium sous atmosphère d'argon on ajoute 475 mL d'un mélage de toluène et d'eau (5/1) prélablement dégazé. Le mélange réactionnel est agité 16 h à 80°C puis refroidi et dilué à l'eau. Après extraction par 2 fois 200 mL de dichlorométhane, les phases organiques réunies sont séchées sur sulfate de sodium, filtrées et concentrées à sec. Le résidu est purifié par chromatographie flash sur une colonne de silice en éluant par des mélanges d'acétate d'éthyle et de méthanol (de 100/0 à 96/4) pour donner 16,1 g de 6-(3-hydroxyméthyl-phényl)-imidazo[1,2-*a*]pyridine-2-carboxylate d'éthyle sous la forme d'un solide jaune clair. Spectre RMN ¹H (DMSO-d6, δ en ppm) : 8,93 (s, 1H), 8,55 (s, 1H), 7,71-7,66 (m, 3H), 7,57 (d, J = 7,7, 1H), 7,48 (t, J = 7,6, 1H), 7,39 (d, J = 7,5, 1H), 5,29 (t, J = 5,7, 1H), 4,61 (d, 5,66, 2H), 4,32 (q, J = 7,1, 2H), 1,34 (t, J = 7,1, 3H).

### Acide 6-(3-hydroxyméthyl-phényl)-imidazo[1,2-a]pyridine-2-carboxylique

A une suspension de 17,9 g de 6-(3-hydroxyméthyl-phényl)-imidazo[1,2-*a*]pyridine-2-carboxylate d'éthyle dans un mélange de 180 mL de tétrahydrofuranne et 9 mL de méthanol, on ajoute 90 mL d'une solution aqueuse 2N de lithine. Le mélange réactionnel est ensuite agité pendant 30 minutes à 20°C. De l'acide chlorhydrique 2N est ajouté goutte à goutte au mélange réactionnel refroidi à 0°C jusqu'à atteindre un pH de 4-5. Le précipité est filtré et lavé deux fois par 50 mL d'éther éthylique pour donner 15,3 g d'acide 6-(3-hydroxyméthyl-phényl)-imidazo[1,2-*a*]pyridine-2-carboxylique sous forme d'un solide blanc.

Spectre RMN ¹H (DMSO-d6, δ en ppm) : 8,97 (s, 1H), 8,52 (s, 1H), 7,77-7,67 (m, 3H), 7,57 (d, J = 7,7, 1H), 7,48 (t, J = 7,6, 1H), 7,39 (d, J = 7,5, 1H), 5,7-4,8 (s large, 1H), 4,60 (s, 2H), (1 H acide peu visible).

Les tableaux qui suivent illustrent les structures chimiques (tableau 1) et les caractéristiques spectroscopiques (tableau 2) de quelques exemples de composés selon l'invention.

**Tableau 1**

| | | | | | |
|---|---|---|---|---|---|
| 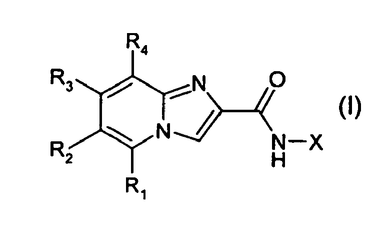 | | | | | |

| **Ex** | **R₁** | **R₂** | **R₃** | **R₄** | **X** |
|---|---|---|---|---|---|
| 01 | H | 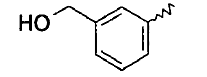 | H | H | 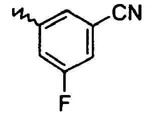 |
| 02 | H | ~NMe₂ | H | H | 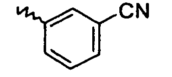 |
| 03 | H | ~NMe₂ | H | H | 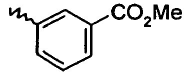 |
| 04 | H | 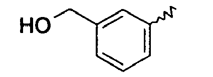 | H | H | 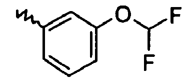 |
| 05 | H | 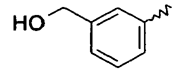 | H | H | 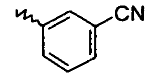 |
| 06 | H | ~NMe₂ | H | H | 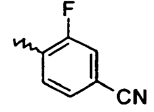 |
| 07 | H | ~NMe₂ | H | H | 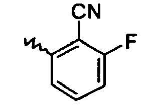 |
| 08 | H | 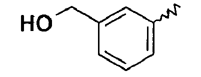 | H | H | 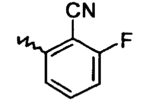 |
| 09 | H | ~NMe₂ | H | H | 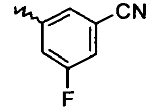 |

**Tableau 2**

| **Ex** | **Caractérisations** |
|---|---|
| **01** | Spectre RMN 1H (DMSO-d6, δ en ppm) : 4,61 (d large, J = 5,5 Hz, 2H), 5,29 (t large, J = 5,5 Hz, 1H), 7,40 (d large, J = 7,5 Hz, 1H), 7,49 (t, J = 7,5 Hz, 1H), de 7,52 à 7,63 (m, 2H), 7,69 (s large, 1H), 7,77 (m, 2H), 8,21 (td, J = 1,5 et 11,5 Hz, 1H), 8,28 (t, J = 1,5 Hz, 1H), 8,60 (s, 1H), 9,00 (t, J = 1,5 Hz, 1H), 10,95 (s, 1H). Spectre de masse (LC-MS-DAD-ELSD) : m/z 387 [M+H]⁺. |
| **02** | Spectre RMN 1H (DMSO-d6, δ en ppm) : 2,87 (s, 6H), 7,36 (dd, J=9,9, 2,5 Hz, 1H), 7,45 - 7,60 (m, 3H), 7,89 (dd, J=2,5, 1,1 Hz, 1H), 8,21 (dt, J=7,1, 2,2 Hz, 1H), 8,33 - 8,41 (m, 2H), 10,56 (s, 1H). Spectre de masse (LC-MS-DAD-ELSD) : m/z 304 [M-H]⁻, m/z 306 [M+H]⁺. |
| **03** | Spectre RMN 1H (DMSO-d6, δ en ppm) : 2,87 (s, 6H), 3,87 (s, 3 H), 7,36 (dd, J=10,0, 2,4 Hz, 1H), 7,47 (t, J=8,0 Hz, 1H), 7,51 (dt, J=10,0, 0,8 Hz, 1H), 7,67 (ddd, J=8,0, 2,0, 1,0 Hz, 1H), 7,90 (dd, J=2,4, 0,8 Hz, 1H), 8,06 (ddd, J=8,0, 2,0, 1,0 Hz, 1H), 8,35 (d, J=0,8 Hz, 1H), 8,66 (t, J=2,0 Hz, 1H), 10,40 (s, 1H). Spectre de masse (LC-MS-DAD-ELSD) : m/z 339 [M+H]⁺. |
| **04** | Spectre RMN 1H (DMSO-d6, δ en ppm) : 4,61 (d, J = 5,5 Hz, 2H), 5,29 (t, J = 5,5 Hz, 1H), 6,90 (dd, J = 2,0 et 7,5 Hz, 1H), 7,21 (t, J = 75,0 Hz, 1H), 7,39 (m, 2H), 7,49 (t, J = 7,5 Hz, 1H), 7,60 (d large, J = 7,5 Hz, 1H), 7,69 (s large, 1H), 7,74 (s, 2H), 7,79 (dd, J = 2,0 et 7,5 Hz, 1H), 7,90 (t, J = 2,0 Hz, 1H), 8,55 (s, 1H), 8,99 (t, J = 1,5 Hz, 1H), 10,5 (s, 1H). Spectre de masse (LC-MS-DAD-ELSD) : m/z 410 [M+H]⁺. |
| **05** | Spectre RMN 1H (DMSO-d6, δ en ppm) : 4,60 (d, J = 5,5 Hz, 2H), 5,29 (t, J = 5,5 Hz, 1H), 7,39 (d large, J = 7,5 Hz, 1H), 7,49 (t, J = 7,5 Hz, 1H), de 7,52 à 7,64 (m, 3H), 7,69 (s large, 1H), 7,76 (m, 2H), 8,24 (m, 1H), 8,40 (m, 1H), 8,59 (s, 1H), 9,00 (t, J = 1,5 Hz, 1H), 10,75 (s, 1H). Spectre de masse (LC-MS-DAD-ELSD) : m/z 369 [M+H]⁺. |
| **06** | Spectre RMN 1H (DMSO-d6, δ en ppm) : 2,87 (s, 6H), 7,40 (dd, J=10,0, 2,4 Hz, 1H), 7,57 (dt, J=10,0, 0,9 Hz, 1H), 7,74 (ddd, J=8,4, 2,0, 1,0 Hz, 1H), 7,87 (dd, J=2,4, 0,9 Hz, 1H), 7,97 (dd, J=10,9, 2,0 Hz, 1H), 8,36 - 8,47 (m, 2H) 9,92 (d, J=2,8 Hz, 1H). Spectre de masse (LC-MS-DAD-ELSD) : m/z 322 [M-H]⁻, m/z 324 [M+H]⁺. |
| **07** | Spectre RMN 1H (DMSO-d6, δ en ppm) : 2,88 (s, 6H), 7,30 (m, 1H), 7,39 (dd, J=10,0, 2,4 Hz, 1H), 7,55 (dt, J=10,0, 0,9 Hz, 1H), 7,78 (td, J=8,5, 6,6 Hz, 1H), 7,86 - 7,95 (m, 2H), 8,42 (d, J=0,9 Hz, 1H), 10,40 (s, 1H). Spectre de masse (LC-MS-DAD-ELSD) : m/z 322 [M-H]⁻, m/z 324 [M+H]⁺. |
| **08** | Spectre RMN 1H (DMSO-d6, δ en ppm) : 4,60 (d, J = 5,5 Hz, 2H), 5,29 (t, J = 5,5 Hz, 1H), 7,34 (m, 1H), 7,39 (d large, J = 7,5 Hz, 1H), 7,49 (t, J = 7,5 Hz, 1H), 7,61 (d large, = 7,5 Hz, 1H), 7,70 (s large, 1H), de 7,74 à 7,89 (m, 4H), 8,61 (s, 1H), 9,00 (t, J = 1,5 Hz, 1H), 10,6 (s, 1H). Spectre de masse (LC-MS-DAD-ELSD) : m/z 387 [M+H]⁺. |
| **09** | Spectre RMN 1H (DMSO-d6, δ en ppm) : 2,87 (s, 6H), 7,37 (dd, J=10,0, 2,4 Hz, 1H), 7,46 7,56 (m, 2H), 7,89 (dd, J=2,5, 1,0 Hz, 1H), 8,19 (ddd, J=11,8, 2,8, 2,0 Hz, 1H), 8,24 (t, J=2,0 Hz, 1H), 8,39 (d, J=1,0 Hz, 1H), 10,79 (s, 1H). Spectre de masse (LC-MS-DAD-ELSD) : m/z 322 [M-H]⁻, m/z 324 [M+H]⁺. |

Les composés selon l'invention ont fait l'objet d'essais pharmacologiques permettant de détermine leur effet modulateur sur NOT.

### Evaluation de l'activité in vitro sur cellules N2A

L'activité des composés selon l'invention a été évaluée sur une lignée cellulaire (N2A) exprimant de manière endogène le récepteur de souris Nurr1 et transfectée de manière stable avec l'élément de réponse liant NOT (NBRE) couplé au gène rapporteur luciférase. Les EC₅₀ sont comprises entre 0,01 et 1000 nM. Les essais ont été réalisés selon le mode opératoire décrit ci dessous. La lignée cellulaire Neuro-2A provient de source commerciale standard (ATCC). Le clone Neuro-2A a été obtenu à partir d'une tumeur spontanée provenant d'une souche de souris A albino par R.J Klebe et col. Cette lignée Neuro-2A est ensuite stablement transfectée avec 8NBRE-luciférase. Les cellules N2A-8NBRE sont cultivées jusqu'à confluence dans des flacons de culture de 75 cm² contenant du DMEM supplémenté par 10% de sérum fatal de veau, 4,5 g/L de glucose et 0,4 mg/ml de Généticine. Après une semaine de culture les cellules sont récupérées par de la trypsine 0,25% pendant 30 secondes puis remises en suspension dans du DMEM sans rouge de phénol contenant 4,5g/L de glucose, 10% de sérum délipidé Hyclone et déposées dans des plaques blanches 96 puits à fond transparent. Les cellules sont déposées à raison de 60.000 par puits dans 75 µL pendant 24 heures avant l'addition des produits. Les produits sont appliqués dans 25µL et incubés 24 heures supplémentaires. Le jour de la mesure, on ajoute à chaque puits un volume équivalent (100µL) de Steadylite, puis on attend 30 minutes pour obtenir une lyse complète des cellules et la production maximale du signal. Les plaques sont ensuite mesurées dans un compteur de luminescence pour microplaques après avoir été scellées par un film adhésif. Les produits sont préparés sous forme de solution stock à 10⁻² M, puis dilués dans 100% de DMSO. Chaque concentration de produit est préalablement diluée dans du milieu de culture avant incubation avec les cellules contenant ainsi 0,625% final de DMSO.
Par exemple, les composés n° 5 et 8 ont montré une CE₅₀ de 27 nM et 0,4 nM respectivement.

Il apparaît donc que les composés selon l'invention ont un effet modulateur de NOT.

Les composés selon l'invention peuvent donc être utilisés pour la préparation de médicaments pour leur application en thérapeutique dans le traitement ou la prévention de maladies impliquant les récepteurs NOT.

Ainsi, selon un autre de ses aspects, l'invention a pour objet des médicaments qui comprennent un composé de formule (I), ou un sel d'addition de ce dernier à un acide pharmaceutiquement acceptable.

Selon un autre de ses aspects, l'invention a pour objet des médicaments qui comprennent un composé choisi parmi les composés de formule (I) tels que définis précédemment, ainsi que le N-(3-chloro-4-cyanophényl)-imidazo[1,2-a]pyridine-2-carboxamide, le 3-({[imidazo[1,2-a]pyridine-2-yl]carbonyl}amino)benzoate de méthyle, le 2-({[imidazo[1,2-a]pyridine-2-yl]carbonyl}amino)benzoate de méthyle, et le N-[2-(difluorométhoxy)phényl]-imidazo[1,2-a]pyridine-2-carboxamide, et les sels d'addition de ces composés à un acide pharmaceutiquement acceptable.

Ces médicaments trouvent leur emploi en thérapeutique, notamment dans le traitement et la prévention des maladies neurodégénératives telles que par exemple la maladie de Parkinson, d'Alzheimer, les tauopathies (ex. la paralysie progressive supranucléaire, la démence fronto temporale, la dégénérescence corticobasale, la maladie de Pick); les traumatismes cérébraux comme l'ischémie et les traumatismes crâniens et l'épilepsie ; les maladies psychiatriques comme la schizophrénie, la dépression, la dépendance à une substance, les troubles du déficit de l'attention et de l'hyperactivité ; les maladies inflammatoires du système nerveux central comme la sclérose en plaque, encéphalite, myélite et encéphalomyélite et autres maladies inflammatoires comme les pathologies vasculaires, l'athérosclérose, les inflammations des articulations, l'arthrose, l'arthrite rhumatoïde ; l'ostéoarthrite, la maladie de Crohn, colite ulcereuse; les maladies inflammatoires allergiques telle que l'asthme, les maladies auto-immunes comme le diabète de type 1, lupus, sclérodermies, Syndrome de Guillain-Barré, maladie d'Addison et autres maladies immuno-médiées; l'ostéoporose; les cancers.

Ainsi, la présente invention vise un composé choisi parmi un composé de formule (I) tel que défini précédemment, ainsi que le N-(3-chloro-4-cyanophényl)-imidazo[1,2-a]pyridine-2-carboxamide, le 3-({[imidazo[1,2-a]pyridine-2-yl]carbonyl}amino)benzoate de méthyle, le 2-({[imidazo[1,2-a]pyridine-2-yl]carbonyl}amino)benzoate de méthyle, le N-[2-(difluorométhoxy)phényl]-imidazo[1,2-a]pyridine-2-carboxamide, et les sels d'addition de ces composés à un acide pharmaceutiquement acceptable, pour le traitement de l'un(e) des maladies, troubles ou désordres cités ci-dessus.

Selon un autre de ses aspects, la présente invention concerne l'utilisation d'un composé choisi parmi les composés ci-dessus mentionnés, pour la préparation d'un médicament destiné au traitement et à la prévention de l'un(e) des maladies, troubles ou désordres cités ci-dessus.

Ces composés pourraient être aussi utilisés comme traitement associé à des greffes et/ou transplantations de cellules souches.

Selon un autre de ses aspects, la présente invention concerne des compositions pharmaceutiques comprenant, en tant que principe actif, un composé choisi parmi le groupe de composés défini ci-dessus. Ces compositions pharmaceutiques contiennent une dose efficace d'au moins un composé choisi parmi le groupe de composés défini ci-dessus, ainsi qu'au moins un excipient pharmaceutiquement acceptable.

Lesdits excipients sont choisis selon la forme pharmaceutique et le mode d'administration souhaité, parmi les excipients habituels qui sont connus de l'Homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif, choisi parmi les composés précités, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscarmellose sodique | 6,0 mg |
| Amidon de maïs | 15,0 mg |
| Hydroxypropyl-méthylcellulose | 2,25 mg |
| Stéarate de magnésium | 3,0 mg |

Il peut y avoir des cas particuliers où des dosages plus élevés ou plus faibles sont appropriés. Selon la pratique habituelle, le dosage approprié à chaque patient est déterminé par le médecin selon le mode d'administration, le poids et la réponse dudit patient.

## Revendications

1. Composés de formule (I) : 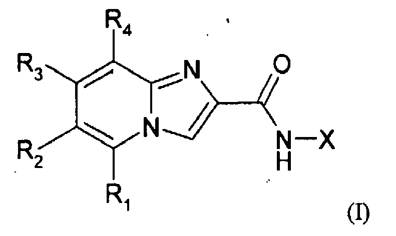 dans laquelle :
X représente un groupe phényle substitué par un cyano, un (C₁-C₆)alcoxycarbonyl, un (C₁-C₆)alcoxy substitué par un ou plusieurs halogènes ou (C₁-C₆)alkyle substitué par un ou plusieurs halogènes, le groupe phényle étant éventuellement substitué une seconde fois par un halogène;
R₁ représente un atome d'hydrogène, un halogène, un groupe (C₁-C₆)alcoxy, un groupe (C₁-C₆)alkyle, un groupe NRaRb; les groupes alkyles et alcoxy pouvant éventuellement être substitués par un ou plusieurs halogène, hydroxy, amino, ou groupe (C₁-C₆)alcoxy,
R₂ représente l'un des groupes suivants :
. un groupe (C₁-C₆)alkyle éventuellement substitué par un ou plusieurs groupes choisis indépendamment les uns des autres parmi un hydroxy, un halogène, un amino, un groupe NRaRb, un groupe (C₁-C₆)alcoxy, un groupe phényle,
. un groupe (C₁-C₆)alcoxy éventuellement substitué par un ou plusieurs groupes, choisis indépendamment les uns des autres parmi hydroxy, halogène, amino, groupe NRaRb,
. un groupe (C₂-C₆)alcényle,
. un groupe (C₂-C₆)alcynyle,
. un groupe -CO-R₅,
. un groupe -CO-NR₆R₇,
. un groupe -CO-O-R₈,
. un groupe -NR₉-CO-R₁₀,
. un groupe -NR₁₁R₁₂,
. un groupe -N=CH-NRaRb,
. un atome d'halogène,
. un groupe cyano, nitro, hydroxyiminoalkyle, alcoxyiminoalkyle,
. un groupe (C₁-C₆)alkylthio,
. un groupe (C₁-C₆)alkylsulfinyle,
. un groupe (C₁-C₆)alkylsulfonyle,
. un groupe (((C₁-C₆)alkyl)₃)silyléthynyle,
. un groupe -SO₂-NR₉R₁₀.
. un groupe phényle éventuellement substitué par un ou plusieurs groupes choisis indépendamment les uns des autres parmi les atomes ou groupes suivants : halogène, (C₁-C₆)alcoxy, cyano, NRaRb, -CO-R₅, -CO-NR₆R₇, -CO-O-R₈, un groupe (C₁-C₆)alkyle éventuellement substitué par un ou plusieurs hydroxy ou NRaRb,
R₃ représente un atome d'hydrogène, un groupe (C₁-C₆)alkyle, un groupe (C₁-C₆)alcoxy ou un atome d'halogène,
R₄ représente un atome d'hydrogène, un groupe (C₁-C₄)alkyle, un groupe (C₁-C₄)alcoxy ou un atome de fluor,
R₅ représente un atome d'hydrogène, un groupe phényle ou un groupe (C₁-C₆)alkyle,
R₆ et R₇, identiques ou différents, représentent un atome d'hydrogène ou un groupe (C₁-C₆)alkyle ou forment avec l'atome d'azote un cycle de 4 à 7 chaînons incluant éventuellement un autre hétéroatome choisi parmi N, O ou S,
R₈ représente un groupe (C₁-C₆)alkyle,
R₉ et R₁₀, identiques ou différents, représentent un atome d'hydrogène ou un groupe (C₁-C₆)alkyle,
R₁₁ et R₁₂ identiques ou différents, représentent un atome d'hydrogène ou un groupe (C₁-C₆)alkyle ou forment avec l'atome d'azote un cycle de 4 à 7 chaînons incluant éventuellement un autre hétéroatome choisi parmi N, O ou S,
Ra et Rb représentent, indépendamment l'un de l'autre, un atome d'hydrogène, un groupe (C₁-C₆)alkyle ou forment avec l'atome d'azote un cycle de 4 à 7 chaînons,
à l'exception des composés où R1 représente un groupe méthyle ou R2 représente un atome de chlore ou R3 représente un groupe méthyle,
à l'état de base ou de sel d'addition à un acide,
à l'exception des composés :
N-(3-chloro-4-cyanophényl)-imidazo[1,2-a]pyridine-2-carboxamide,
3-({[imidazo[1,2-a]pyridine-2-yl]carbonyl}amino)benzoate de méthyle,
2-({[imidazo[1,2-a]pyridine-2-yl]carbonyl}amino)benzoate de méthyle,
N-[2-(difluorométhoxy)phényl]-imidazo[1,2-a]pyridine-2-carboxamide,
N-[3-(trifluorométhoxy)phényl]-6-(diméthylamino)imidazo[1,2-a]pyridine-2-carboxamide,
N-[3-(difluorométhoxy)phényl]-6-(diméthylamino)imidazo[1,2-a]pyridine-2-carboxamide, et
N-[3-(trifluorométhyl)phényl]-6-(diméthylamino)imidazo[1,2-a]pyridine-2-carboxamide.

2. Composé de formule (I) selon la revendication 1, **caractérisé en ce que** :
X représente un groupe phényle substitué par un cyano, un (C₁-C₆)alcoxycarbonyl, un (C₁-C₆)alcoxy substitué par plusieurs halogènes, ledit groupe phényle étant éventuellement substitué une seconde fois par un halogène ;
R₂ représente un groupe-NR₁₁R₁₂ ou un groupe phényle substitué par un groupe (C₁-C₅)alkyle lui-même substitué par un groupe hydroxy ;
R₁, R₃ et R₄ représentent un atome d'hydrogène ;
R₁₁, et R₁₂ identiques ou différents, représentent un groupe (C₁-C₆)alkyle.;
à l'état de base ou de sel d'addition à un acide,
à l'exception des composés où R₁ représente un groupe méthyle ou R₂ représente un atome de chlore ou R₃ représente un groupe méthyle,
à l'exception des composés :
N-[3-(trifluorométhoxy)phényl)-6-(diméthylamino)imidazo[1,2-a]pyridine-2-carboxamide et
N-[3-(difluorométhoxy)phényl)-6-(diméthylamino)imidazo[1,2-a]pyridine-2-carboxamide.

3. Composé de formule (I) selon l'une quelconque des revendications précédentes, **caractérisé en ce que** :
X représente un groupe phényle substitué par un cyano, CO₂Me, -OCHF₂, éventuellement substitué une seconde fois par un atome de fluor ;
R₁, R₃ et R₄ représentent un atome d'hydrogène ;
R₂ représente un groupe N-diméthyle, ou un groupe phényle substitué par un groupe hydroxyméthyle,
à l'état de base ou de sel d'addition à un acide,
à l'exception du N-[3-(difluorométhoxy)phényl]-6-(diméthyiamino)imidazo[1,2-a]pyridine-2-carboxamide.

4. Composé selon l'une quelconque des revendications précédentes, **caractérisé en ce qu'**il est choisi parmi :
• *N*-(3-cyano-5-fluorophényl)-6-[3-(hydroxyméthyl)phényl]imidazo[1,2-*a*]pyridine-2-carboxamide
• *N*(3-cyanophényl)-6-(diméthylamino)imidazo[1,2-*a*]pyridine-2-carboxamide
• 3-({[6-(Diméthylamino)imidazo[1,2-*a*]pyridin-2-yl]carbonyl}amino)benzoate de méthyle
• *N*-[3-(difluorométhoxy)phényl]-6-[3-(hydroxyméthyl)phényl]imidazo[1,2-*a*]pyridine-2-carboxamide
• *N*-(3-cyanophényl)-6-[3-(hydroxyméthyl)phényl]imidazo[1,2-*a*]pyridine-2-carboxamide
• *N*-(4-cyano-2-fluorophényl)-6-(diméthylamino)imidazo[1,2-*a*]pyridine-2-carboxamide
• *N*-(2-cyano-3-fluorophényl)-6-(diméthylamino)imidazo[1,2-*a*]pyridine-2-carboxamide
• *N*-(2-cyano-3-fluorophényl)-6-[3-(hydroxyméthyl)phényl]imidazo[1,2-*a*]pyridine-2-carboxamide
• *N*-(3-cyano-5-fluorophényl)-6-(diméthylamino)imidazo[1,2-*a*]pyridine-2-carboxamide

5. Médicament, **caractérisé en ce qu'**il comprend un composé choisi parmi les composés de formule (1) selon l'une quelconque des revendications 1 à 4, ainsi que le N-(3-chloro-4-cyanophényl)-imidazo[1,2-a]pyridine-2-carboxamide, le 3-({[imidazo[1,2-a]pyridine-2-yl]carbonyl}amino)benzoate de méthyle, le 2-({[imidazo[1,2-a]pyridine-2-yl]carbonyl}amino)benaoate de méthyle, le N-[2-(difluoromethoxy)phényl]-imidazo[1,2-a]pyridine-2-carboxamide, et les sels d'addition de ces composés à un acide pharmaceutiquement acceptable.

6. Médicament, **caractérisé en ce qu'**il comprend un composé de formule (I) selon l'une quelconque des revendications 1 à 4, ou un sel d'addition de ces composés à un acide pharmaceutiquement acceptable.

7. Composition pharmaceutique, **caractérisée en ce qu'**elle comprend un composé tel que défini selon l'une quelconque des revendications 5 ou 6, ainsi qu'au moins un excipient pharmaceutiquement acceptable.

8. Utilisation d'un composé tel que défini selon l'une quelconque des revendications 5 ou 6, pour la préparation d'un médicament destiné au traitement et à la prévention des maladies neurodégénératives.

9. Utilisation d'un composé tel que défini selon l'une quelconque des revendications 5 où 6, pour la préparation d'un médicament destiné au traitement et à la prévention des traumatismes cérébraux et de l'épilepsie.

10. Utilisation d'un composé tel que défini selon l'une quelconque des revendications 5 ou 6, pour la préparation d'un médicament destiné au traitement et à la prévention des maladies psychiatriques.

11. Utilisation d'un composé tel que défini selon l'une quelconque des revendications 5 ou 6, pour la préparation d'un médicament destiné au traitement et à la prévention des maladies inflammatoires.

12. Utilisation d'un composé tel que défini selon l'une quelconque des revendications 5 ou 6, pour la préparation d'un médicament destiné au traitement et à la prévention de l'ostéoporose et les cancers.

13. Utilisation d'un composé tel que défini selon l'une quelconque des revendications 5 ou 6, pour la préparation d'un médicament destiné au traitement et à la prévention de la maladie de Parkinson, d'Alzheimer, des tauopathies, de la sclérose en plaque.

14. Utilisation d'un composé tel que défini selon l'une quelconque des revendications 5 ou 6, pour la préparation d'un médicament destiné au traitement et à la prévention de la schizophrénie, la dépression, la dépendance à une substance les troubles du déficit de l'attention et de l'hyperactivité.

15. Composés :
Acide 6-diméthylamino-imidazo[1,2-*a*]pyridine-2-carboxylique
6-(3-Hydroxyméthyl-phényl)-imidazo[1,2-*a*]pyridine-2-carboxylate d'éthyle
Acide 6-(3-hydroxyméthyl-phényl)-imidazo[1,2-*a*]pyridine-2-carboxylique.

16. Utilisation des composés selon la revendication 15 pour la synthèse de produits de formule générale (1) telle que définie dans la revendication 1.

## Claims

1. Compound of formula (I): 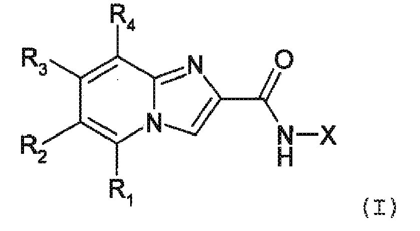 in which:
X represents a phenol group substituted by a cyano, a (C₁-C₆)alkoxycarbonyl, a (C₁-C₆)alkoxy substituted by one or more halogens or (C₁-C₆)alkyl substituted by one or more halogens, the phenyl group optionally being substituted a second time by a halogen;
R₁ represents a hydrogen atom, a halogen, a (C₁- C₆)alkoxy group, a (C₁-C₆)alkyl group or an NRaRb group, it being possible for the alkyl and alkoxy groups to be optionally substituted by one or more halogen, hydroxyl, amino, or (C₁-C₆)alkoxy group;
R₂ represents one of the following groups:
. a(C₁-C₆)alkyl group optionally substituted by one or more groups chosen, independently of one another, from a hydroxyl, a halogen, an amino, an NRaRb group, a (C₁-C₆)alkoxy group or a phenyl group,
. a (C₁-C₆)alkoxy group optionally substituted by one or more groups chosen, independently of one another, from hydroxyl, halogen, amino or NRaRb group,
. a (C₂-C₆)alkenyl group,
. a (C₂-C₆)alkynyl group,
. a -CO-R₅ group,
. a -CO-NR₆R₇ group,
. a -CO-O-R₈ group,
. an -NR₉-CO-R₁₀ group,
. an -NR₁₁R₁₂ group,
. an -N=CH-NRaRb group,
. a halogen, atom,
. a cyano, nitro, hydroxyiminoalkyl or an alkoxyiminoalkyl group,
. a (C₁-C₆)alkylthio group,
. a (C₁-C₆)alkylsulphinyl group,
. a (C₁-C₆)alkylsulphonyl group,
. a ((C₁-C₆)alkyl)₃silylethynyl group,
. an -SO₂-NR₉R₁₀ group,
. a phenyl group optionally substituted by one or more groups chosen, independently of one another, from the following atoms or groups: halogen, (C₁-C₆)alkoxy, cyano, NRaRb, -CO-R₅, -CO-NR₆R₇, -CO-O-R₈ or a (C₁-C₆)alkyl group optionally substituted by one or more hydroxyl or NRaRb groups;
R₃ represents a hydrogen atom, a (C₁-C₆)alkyl group, a (C₁-C₆)alkoxy group or a halogen, atom;
R₄ represents a hydrogen atom, a (C₁-C₄)alkyl group, a (C₁-C₄)alkoxy group or a fluorine atom;
R₅ represents a hydrogen atom, a phenyl group or a (C₁- C₆) alkyl group; a
R₆ and R₇, which are identical or different, represent a hydrogen atom or a (C₁-C₆)alkyl group or form, with the nitrogen atom, a 4- to 7-membered ring optionally including another heteroatom chosen from N, O or S;
R₈ represents a (C₁-C₆)alkyl group;
R₉ and R₁₀, which are identical or different, represent
a hydrogen atom or a (C₁-C₆)alkyl group;
R₁₁ and R₁₂, which are identical or different, represent a hydrogen atom or a (C₁-C₆)alkyl group or form, with the nitrogen atom, a 4- to 7-membered ring optionally including another heteroatom chosen from N, O or S;
Ra and Rb represent, independently of one another, a hydrogen atom or a (C₁-C₆)alkyl group or form, with the nitrogen atom, a 4- to 7-membered ring;
with the exception of the compounds where R₁ represents a methyl group or R₂ represents a chlorine atom or R₃ represents a methyl group,
in the form of the base or of an addition salt with an acid,
with the exception of the compounds:
N-(3-chloro-4-cyanophenyl)imidazo[1,2-a]pyridine-2-carboxamide,
methyl 3-({[imidazo[1,2-a]pyridin-2-yl]carbonyl}amino)benzoate,
methyl 2-({[imidazo[1,2-a]pyridin-2-yl]carbonyl}amino)benzoate,
N-[2-(difluoromethoxy)phenyl]imidazo[1,2-a]pyridine-2-carboxamide,
N-[3-(trifluoromethoxy)phenyl]-6-(dimethylamino)imidaso[1,2-a]pyridine-2-carboxamide,
N-[3-(difluoromethoxy)phenyl]-6-(dimethylamino)imidazo[1,2-a]pyridine-2-carboxamide, and
N-[3-(trifluoromethyl)phenyl]-6-(dimethylamino)imidazo[1,2-a]pyridine-2-carboxamide.

2. Compound of formula (I) according to Claim 1, **characterized in that**:
X represents a phenyl group substituted by a cyano, a (C₁-C₆)alkoxycarbonyl or a (C₁-C₆)alkoxy substituted by several halogens, the said phenyl group being optionally substituted a second time by a halogen;
R₂ represents an -NR₁₁R₁₂ group or a phenol group substituted by a (C₁-C₆)alkyl group, itself substituted by a hydroxyl group;
R₁, R₃ and R₄ represent a hydrogen atom;
R₁₁ and R₁₂, which are identical or different, represent a (C₁-C₆)alkyl group;
in the form of the base or of an addition salt with an acid,
with the exception of the compounds where R₁ represents a methyl group or R₂ represents a chlorine atom or R₃ represents a methyl group,
with the exception of the compounds:
N-[3-(trifluoromethoxy)phenyl]-6-(dimethylamino)imidazo[1,2-a]pyridine-2-carboxamide, and
N-[3-(difluoromethoxy)phenyl]-6-(dimethylamino)imidazo[1,2-a]pyridine-2-carboxamide.

3. Compound of formula (I) according to one of the preceding claims, **characterized in that**:
X represents a phenyl group substituted by a cyano,
Come or -OCHF₂ and optionally substituted a second time by a fluorine atom;
R₁, R₃ and R₄. represent a hydrogen atom;
R₂ represents an N-dimethyl group or a phenyl group substituted by a hydroxymethyl group;
in the form of the base or of an addition salt with an acid,
with the exception of N-[3-(difluoromethoxy)phenyl]-6-(dimethylamino)imidazo[1,2-a]pyridine-2-carboxamide,

4. Compound according to any one of the preceding claims, **characterized in that** it is chosen from:
• *N*-(3-cyano-5-fluorophenyl)-6-[3-(hydroxymethyl)phenyllimidazo[1,2-*a*]pyridine-2-carboxamide,
• *N*-(3-cyanophenyl)-6-(dimethylamino)imidazo[1,2-*a*]pyridine-2-carboxamide,
• Methyl 3-({[6-(dimethylamino)imidazo[1,2-*a*]pyridin-2-yl]carbonyl}amino)benzoate,
• *N*-[3-(difluoromethoxy)phenyl]-6-[3-(hydroxymethyl)phenyl]imidazo[1,2-*a*]pyridine-2-carboxamide,
• *N*-(3-cyanophenyl)-6-[3-(hydroxylmethyl)phanyl]imidazo[1,2-*a*]pyridine-2-carboxamide,
• *N*-(4-cyano-2-fluorophenyl)-6-(dimethylamino)imidazo[1,2-*a*]pyridine-2-carboxamide,
• *N*-(2-cyano-3-fluorophenyl)-6-(dimethylamino)imidazo[1,2-*a*]pyridine-2-carboxamide,
• *N*-(2-cyano-3-fluorophenyl)-6-[3-(hydroxymethyl)phenyl]imidazo[1,2-*a*]pyridine-2-carboxamide,
• *N*-(3-cyano-5-fluorophenyl)-6-(dimethylamino)imidazo[1,2-*a*]pyridine-2-carboxamide.

5. Medicament, **characterized in that** it comprises a compound chosen from the compounds of formula (I) according to any one of Claims 1 to 4, and also N-(3-chloro-4-cyanophenyl)imidazo[1,2-a]pyridine-2-carboxamide, methyl 3-({[imidazo[1,2-a]pyridin-2-yl]carbonyl}amino)benzoate, methyl 2-({[imidazo[1,2-alpyridin-2-yl]carbonyl}amino)benzoate and N-[2-(diiluoromethoxy)phenyllimidazo[1,2-a]pyridine-2-carboxamide, and the addition salts of these compounds with a pharmaceutically acceptable acid.

6. Medicament, **characterized in that** it comprises a compound of formula (I) according to any one of Clams 1 to 4, or an addition salt of these compounds with a pharmaceutically acceptable acid.

7. Pharmaceutical composition, **characterized in that** it comprises a compound as defined according to either one of Claims 5 and 6, and also at least one pharmaceutically acceptable excipient.

8. Use of a compound as defined according to either one of Claims 5 and 6 in the preparation of a medicament intended for the treatment and prevention of neurodegenerative diseases.

9. Use of a compound as defined according to either one of Claims 5 and 6 in the preparation of a medicament intended for the treatment and prevention of cerebral traumas and epilepsy.

10. Use of a compound as defined according to either one of Claims 5 and 6 in the preparation of a medicament intended for the treatment and prevention of psychiatric diseases.

11. Use of a compound as defined according to either one of Claims 5 and 6 in the preparation of a medicament intended for the treatment and prevention of inflammatory diseases.

12. Use of a compound as defined according to either one of Claims 5 and 6 in the preparation of a medicament intended for the treatment and prevention of osteoporosis and cancers.

13. Use of a compound as defined according to either one of Claims 5 and 6 in the preparation of a medicament intended for the treatment and prevention of Parkinson's disease, Alzheimer's disease, tauopathies and multiple sclerosis.

14. Use of a compound as defined according to either one of Claims 5 and 6 in the preparation of a medicament intended for the treatment and prevention of schizophrenia, depression, substance dependence and attention deficit hyperactivity disorders.

15. Compounds:
6-dimethylaminoimidazo[1,2*-a*]pyridine-2-carboxylic acid Ethyl 6-[3-(hydroxymethyl)phenyl]imidazo[1,2-a]pyridine-2-carboxylate
6-[3-(hydroxymethyl)phenyl]imidazo[1,2*-a*]pyridine-2-carboxylic acid.

16. Use of the compounds according to Claim 15 in the synthesis of products of general formula (I) as defined in Claim 1.

## Patentansprüche

1. Verbindungen der Formel (I): 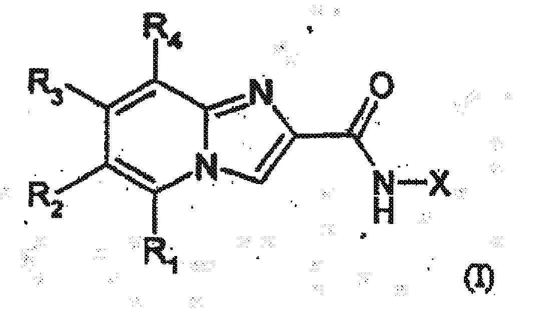 worin:
X für eine Phenylgruppe, die durch ein Cyano, ein (C₁-C₆)-Alkoxycarbonyl, ein (C₁-C₆)-Alkoxy, das durch ein oder mehrere Halogene substituiert ist,
oder ein (C₁-C₆)-Alkyl, das durch ein oder mehrere Halogene substituiert ist, substituiert ist, steht, wobei die Phenylgruppe gegebenenfalls ein zweites Mal durch ein Halogen substituiert ist;
R₁ für ein Wasserstoffatom, ein Halogen, eine (C₁-C₆)-Alkoxygruppe, eine (C₁-C₆) -Alkylgruppe oder' eine NRaRb-Gruppe steht; wobei die Alkyl- und
Alkoxygruppen gegebenenfalls durch ein bzw. eine oder mehrere Halogene, Hydroxy, Amino oder (C₁-C₆)-Alkoxygruppen substituiert sein können,
R₂ für eine der folgenden Gruppen steht:
• eine (C₁-C₆) -Alkylgruppe, die gegebenenfalls durch eine oder mehrere Gruppen, die unabhängig voneinander unter Hydroxy, Halogen, Amino, einer NRaRb-Gruppe, einer (C₁-C₆) -Alkoxygruppe und einer Phenylgruppe ausgewählt sind, substituiert ist,
• eine (C₁-C₆)-Alkoxygruppe, die gegebenenfalls durch eine oder mehrere Gruppen, die unabhängig voneinander unter Hydroxy, Halogen, Amino und einer NRaRb-Gruppe ausgewählt sind, substituiert ist,
• eine (C₂-C₆)-Alkenylgruppe,
• eine (C₂-C₆)-Alkinylgruppe,
• eine -CO-R₅-Gruppe,
• eine -CO-NR₆R₇-Gruppe,
• eine -CO-O-R₈-Gruppe,
• eine -NR₉-CO-R₁₀)-Gruppe,
• eine -NR₁₁R₁₂-Gruppe,
• eine -N=CH-NRaRb-Gruppe,
• ein Halogenatom,
• eine Cyano-, Nitro-, Hydroxyiminoalkyl- oder Alkoxyiminoalkylgruppe,
• eine (C₁-C₆) -Alkylthiogruppe,
• eine (C₁-C₆)-Alkylsulfinylgruppe,
• eine (C₁-C₆) -Alkylsulfonylgruppe,
• eine (C₁-C₆) -Alkyl)₃) silylethinylgruppe,
• eine -SO₂-NR₉R₁₀-Gruppe,
• eine Phenylgruppe, die gegebenenfalls durch eine oder mehrere Gruppen substituiert ist, die unabhängig voneinander unter den folgenden Atomen bzw. Gruppen ausgewählt sind: Halogen, (C₁-C₆)-Alkoxy, Cyano, NRaRb, -CO-R₅, -CO-NR₆R₇, -CO-O-R₈ und einer (C₁-C₆) -Alkylgruppe, die gegebenenfalls durch ein oder mehrere Hydroxy oder NRaRb substituiert ist,
R₃ für ein Wasserstoffatom, eine (C₁-C₆)-Alkylgruppe, eine (C₁-C₆) -Alkoxygruppe oder ein Halogenatom steht,
R₄ für ein Wasserstoffatom, eine (C₁-C₄)-Alkylgruppe, eine (C₁-C₄)-Alkoxygruppe oder ein Fluoratom steht,
R₅ für ein Wasserstoffatom, eine Phenylgruppe oder
eine (C₁-C₆)-Alkylgruppe steht,
R₆ und R₇ gleich oder verschieden sind und für ein Wasserstoffatom oder eine (C₁-C₆)-Alkylgruppe stehen oder mit dem Stickstoffatom einen 4- bis 7-gliedrigen Ring, der gegebenenfalls ein weiteres unter N, O und S ausgewähltes Heteroatom enthält, bilden,
R₈ für eine (C₁-C₆)-Alkylgruppe steht,
R₉ und R₁₀ gleich oder verschieden sind und für ein Wasserstoffatom oder eine (C₁-C₆) -Alkylgruppe stehen,
R₁₁ und R₁₂ gleich der verschieden sind und für ein Wasserstoffatom oder eine (C₁-C₆) -Alkylgruppe stehen oder mit dem Stickstoffatom einen 4- bis 7-gliedrigen Ring, der gegebenenfalls ein weiteres unter N, O und S ausgewähltes Heteroatom enthält, bilden,
Ra und Rb unabhängig voneinander für ein Wasserstoffatom oder eine (C₁-C₆) -Alkylgruppe stehen oder mit dem Stickstoffatom einen 4- bis 7-gliedrigen Ring bilden,
mit Ausnahme der Verbindungen, in denen R₁ für eine Methylgruppe steht oder R₂ für ein Chloratom steht oder R₃ für eine Methylgruppe steht,
in Basen- oder Säureadditionssalzform,
mit Ausnahme der folgenden Verbindungen:
N-(3-Chlor-4-cyanophenyl) imidazo [1,2-a] pyridin-2-carboxamid,
3-({[Imidazo[1,2-a]pyridin-2-yl]carbonyl}amino) benzoesäuremethylester,
2-({[Imidazo[1,2-a]pyridin-2-yl]carbonyl}amino)-benzoesäuremethylester,
N-[2-(Difluormethoxy)phenyl]imidazo[1,2-a]pyridin-2-carboxamid,
N-[3-(Trifluormethoxy)phenyl]-6-(dimethylamino)-imidazo[1,2-a]pyridin-2--carboxamid,
N-[3-(Difluormethoxy)phenyl]-6-(dimethylamino)-imidazo[1,2-a]pyridin-2-carboxamid und N-[3-(Trifluormethyl)phenyl]-6-(dimethylamino)-imidazo[1,2-a]pyridin-2-carboxamid.

2. Verbindung der Formel (I) nach Anspruch 1, **dadurch gekennzeichnet, daß**:
X für eine Phenylgruppe, die durch ein Cyano, ein (C₁-C₆)-Alkoxycarbonyl oder ein (C₁-C₆) -Alkoxy, das durch mehrere Halogene substituiert ist,
substituiert ist, steht, wobei die Phenylgruppe gegebenenfalls ein zweites Mal durch ein Halogen substituiert ist;
R₂ für eine -NR₁₁R₁₂-Gruppe oder eine Phenylgruppe,
die durch eine (C₁-C₆) -Alkylgruppe substituiert ist, die selbst durch eine Hydroxygruppe substituiert ist, steht;
R₁, R₃ und R₄ für ein Wasserstoffatom stehen;
R₁₁ und R₁₂ gleich oder verschieden sind und für eine (C₁-C₆) -Alkylgruppe stehen;
in Basen- oder Säureadditionssalzform,
mit Ausnahme der Verbindungen, in denen R₁ für eine
Methylgruppe steht oder R₂ für ein Chloratom steht oder R₃ für eine Methylgruppe steht,
mit Ausnahme der folgenden Verbindungen:
N-[3-(Trifluormethoxy)phenyl]-6-(dimethylamino)-imidazo[1,2-a]pyridin-2-carboxamid und
N-[3-(Difluormethoxy)phenyl]-6-(dimethylamino) imidazo[1,2-a]pyridin-2-carboxamid.

3. Verbindung der Formel (I) nach einem der vorhergehenden Ansprüche, **dadurch gekennzeichnet, daß**:
X für eine Phenylgruppe, die durch ein Cyano, CO₂Me oder -OCHF₂ substituiert ist und gegebenenfalls ein zweites Mal durch ein Fluoratom substituiert ist, steht;
Rᵢ₁, R₃ und F₄ für ein Wasserstoffatom stehen;
R₂ für eine N-Dimethylgruppe oder eine Phenylgruppe, die durch eine Hydroxymethylgruppe substituiert ist, steht;
in Basen- oder Säureadditionssalzform,
mit Ausnahme von N-[3-(Difluormethoxy)phenyl] -6-(dimethylamino)imidazo[1,2-a]pyridin-2-carboxamid.

4. Verbindung nach einem der vorhergehenden Ansprüche, **dadurch gekennzeichnet, daß** sie unter:
• *N*-(3-Cyano-5-fluorphenyl)-6-[3-(hydroxymethyl)-phenyl]imidazo[1,2*-a*]pyridin-2-carboxamid
• *N-*(3-Cyanophenyl)-6-(dimethylamino)imidazo[1,2-*a*] pyridin-2-carboxamid
• 3-({[6-(Dimethylamino)imidazo[1,2-*a*]pyridin-2-yl]carbonyl}amino)benzoesäuremethylester
• *N-*[3-(Difluormethoxy)phenyl]-6-[3-(hydroxymethyl)phenyl]imidazo[1,2*-a*]pyridin-2-carboxamid
• *N-*(3-Cyanophenyl)-6-[3-(hydroxymethyl)phenyl]-imidazo[1,2*-a*]pyridin-2-carboxamid
• *N-*(4-Cyano-2-fluorphenyl)-6-(dimethylamino) imidazo[1,2*-a*]pyridin-2-carboxamid
• *N-*(2-Cyano-3-fluorphenyl)-6-(dimethylamino) imidazo[1,2*-a*]pyridin-2-carboxamid
• *N-*(2-Cyano-3-fluorphenyl)-6-[3-(hydroxymethyl) phenyl]imidazo[1,2*-a*]pyridin-2-carboxamid
• *N-*(3-Cyano-5-fluorphenyl)-6-(dimethylamino)-imidazo[1,2*-a*]pyridin-2-carboxamid ausgewählt ist.

5. Medikament, **dadurch gekennzeichnet, daß** es eine unter den Verbindungen der Formel (I) nach einem der Ansprüche 1 bis 4 sowie N-(3-Chlor-4-cyanophenyl)imidazo[1,2-a]pyridin-2-carboxamid, 3-({[Imidazo[1,2-a]pyridin-2-yl]carbonyl}amino)-benzoesäuremethylester, 2-({[Imidazo[1,2-a]-pyridin-2-yl]carbonyl}amino)benzoesäuremethylester und N-[2-(Difluormethoxy)phenyl]imidazo[1,2-a]-pyridin-2-carboxamid und den Additionssalzen dieser Verbindungen mit einer pharmazeutisch unbedenklichen Säure ausgewählte Verbindung enthält.

6. Medikament, **dadurch gekennzeichnet, daß** es eine Verbindung der Formel (I) nach einem der Ansprüche 1 bis 4 oder ein Additionssalz dieser Verbindungen mit einer pharmazeutisch unbedenklichen Säure enthält.

7. Pharmazeutische Zusammensetzung, **dadurch gekennzeichnet, daß** sie eine Verbindung gemäß einem der Ansprüche 5 und 6 sowie mindestens einen pharmazeutisch unbedenklichen Hilfsstoff enthält.

8. Verwendung einer Verbindung gemäß einem der Ansprüche 5 und 6 zur Herstellung eines Medikaments zur Behandlung und Prävention von neurodegenerativen Erkrankungen.

9. Verwendung einer Verbindung gemäß einem der Ansprüche 5 und 6 zur Herstellung eines Medikaments zur Behandlung und Prävention von Hirntraumen und Epilepsie.

10. Verwendung einer Verbindung gemäß einem der Ansprüche 5 und 6 zur Herstellung eines Medikaments zur Behandlung und Prävention von psychiatrischen Erkrankungen.

11. Verwendung einer Verbindung gemäß einem der Ansprüche 5 und 6 zur Herstellung eines Medikaments zur Behandlung und Prävention von entzündliche Erkrankungen.

12. Verwendung einer Verbindung gemäß einem der Ansprüche 5 und 6 zur Herstellung eines Medikaments zur Behandlung und Prävention von Osteoporose und Krebserkrankungen.

13. Verwendung einer Verbindung gemäß einem der Ansprüche 5 und 6 zur Herstellung eines Medikaments zur Behandlung und Prävention von Parkinson-Krankheit, Alzheimer-Krankheit Tauopathien und multipler Sklerose.

14. Verwendung einer Verbindung gemäß einem der Ansprüche 5 und 6 zur Herstellung eines Medikaments zur Behandlung und Prävention von Schizophrenie, Depression, Substanzabhängigkeit und Aufmerksamkeitsdefizit-Hyperaktivitätssyndrom.

15. Verbindungen:
6-Dimethylaminoimidazo[1,2*-a*]pyridin-2-carbonsäure
6-[3-(Hydroxymethyl)phenyl]imidazo[1,2*-a*]pyridin-2-carbonsäureethylester
6-[3-(Hydroxymethyl)phenyl]imidazo[1,2*-a*]pyridin-2-carbonsäure.

16. Verwendung der Verbindungen nach Anspruch 15 zur Herstellung von Produkten der allgemeinen Formel (I) gemäß Anspruch 1,
